# EUROPÄISCHE PATENTSCHRIFT

(11) **EP 0 812 819 B1**
(45) Veröffentlichungstag und Bekanntmachung des Hinweises auf die Patenterteilung: **28.11.2001**
(21) Anmeldenummer: 97106223.7
(22) Anmeldetag: 16.04.1997
(51) Int. Cl.: C07C 69/34, C12P 41/00, C12P 7/62

(54) **Enantiomerenangereicherte , durch einen tertiären Kohlenwasserstoffrest substituierte Malonsäuremonoester sowie deren Herstellung**
Enantiomer-enriched tertiary hydrocarbonrest-substituted malonic acid ester and the preparation
Enantiomère enrichie d'un ester de l'acide malonique substitué par des restes d'hydrocarbures tertiaires et sa préparation

(30) Priorität: 10.06.1996 DE 19623142
(43) Veröffentlichungstag der Anmeldung: 17.12.1997
(73) Patentinhaber: Degussa AG, 40474 Düsseldorf (DE)
(72) Erfinder: Jegelka, Udo, Dr., 45657 Recklinghausen (DE); Kleemiss, Wolfgang, Dr., 45721 Haltern (DE); Klotz--Berendes, Bruno, Dr., 59368 Werne (DE); Feld, Marcel, Dr., 51147 Köln (DE)

(56) Entgegenhaltungen:
- EP-A- 0 050 799
- SE-B- 453 599
- KITAZUME, TOMOYA ET AL: "Microbial approach to the practical monofluorinated chiral synthons" J. ORG. CHEM. (1986), 51(7), 1003-6 , XP002114665
- REGIS LEUNG-TOUNG ET AL.: "Flash vacuum pyrolysis of tert-butyl beta-ketoesters:Sterically protected alpha oxoketenes" TETRAHEDRON., Bd. 48, Nr. 36, 1992, Seiten 7641-7654, XP002114666 OXFORD GB
- DATABASE WPI Section Ch, Week 8224 Derwent Publications Ltd., London, GB; Class B05, AN 82-004555 XP002114667 & HU 22 369 A (RICHTER GEDEON VEGYESZETI GYAR), 28. Mai 1982 (1982-05-28)
- KLOTZ-BERENDES, BRUNO ET AL: "Enzymic synthesis of optically active monoalkylated malonic monoesters" TETRAHEDRON: ASYMMETRY (1997), 8(11), 1821-1823 , XP002114677

## Beschreibung

Die Erfindung betrifft enantiomerenangereicherte, durch einen tertiären Kohlenwasserstoffrest substituierte Malonsäuremonoester und deren Salze sowie ein Verfahren zu deren Herstellung aus den entsprechenden prochiralen Malonsäurediestern durch enzymatische Teilhydrolyse (oder partielle Verseifung).

Enantiomerenangereicherte Verbindungen haben eine erhebliche Bedeutung als Synthesebausteine im Pharma- und im Agrobereich sowie als chirale Auxiliare. Als Ausgangsstoffe für enantiomerenangereicherte α-mono- oder α,α-disubstituierte Malonsäuremonoester bieten sich die entsprechenden Diester an, die ein prochirales Kohlenstoffatom mit zwei gleichen Carbonestergruppen und zwei weiteren. voneinander und von den Carbonestergruppen verschiedene Substituenten enthalten. Durch Teilverseifung zum Monoester entsteht ein Chiralitatszentrum, und eine enantioselektive Teilhydrolyse ergibt enantiomerenangereicherte und im günstigen Fall praktisch enantiomerenreine Malonsäuremonoester.

α,α-disubstituierte Malonsäurediester können mit Hilfe von Schweineleberesterase selektiv in enantiomerenangereicherte Malonsäuremonoester überführt werden (M.Schneider et al.. Angew. chem. 96 [1984]. 54). Dabei erhält man gute Enantiomerenüberschüsse, wenn der Größenunterschied der Substituenten ausgeprägt ist. Auch α-Chymotrypsin ist ein brauchbares Enzym, um α,α-Dialkylmalonsäurediester enantioselektiv zu den entsprechenden α,α-Dialkylmalonsäuremonoestern partiell zu verseifen (F.Bjorkling et al . Tetrahedron, **41** [1985]. 1347).

Diese bekannte enzymkatalysierte Teilhydrolyse konnte nicht auf α-Monoalkylmalonsäurediester übertragen werden. T.Kitazume et al. (J.Org. Chem. **51** [1986], 1003) haben keine nennenswerten Enantiomerenüberschüsse erzielen können, da die betreffenden Monoester unter den Reaktions- bzw. Aufarbeitungsbedingungen sofort racemisierten.

A.L.Gutman et al. (J.Org.Chem. **57** [1992], 1063) haben aus α-Methoxymalonsäuredimethylester durch Umesterung mit Benzylalkohol bei Verwendung von Lipase aus Candida cylindracea als Umesterungskatalysator α-Methoxymalonsäurebenzylmethylester mit einem Enantiomerenüberschuß von 98% erhalten. Durch katalytische Hydrierung konnte daraus der α-Methoxymalonsäuremonomethylester gewonnen werden. Diese Arbeitsweise ist auf die Methoxyverbindung beschränkt und als zweistufiges Verfahren relativ aufwendig. Außerdem ist der Monoester nur in organischen Lösungsmitteln konfigurationsstabil.

Durch die Erfindung werden nun enantiomerenangereicherte, durch einen tertiären Kohlenwasserstoffrest α-monosubstituierte Malonsäuremonoester oder deren Salze der allgemeinen Formel I: 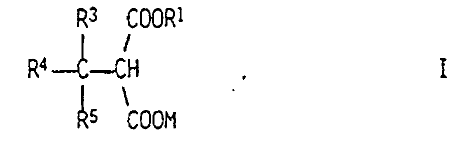 bereitgestellt, in der R¹, R³, R⁴ und R⁵ gleiche oder verschiedene Kohlenwasserstoffreste bedeuten, jeweils zwei der Reste R³, R⁴ und R⁵ zusammen mit dem quartären Kohlenstoffatom, das sie substituieren. auch einen carbocyclischen Ring bilden können und M Wasserstoff, ein Metalläquivalent oder ein gegebenenfalls substituiertes Ammonium-Ion bezeichnet.

Durch die Erfindung wird weiterhin ein Verfahren zur Herstellung der enantiomerenangereicherten, durch einen tertiären Kohlenwasserstoffrest α-monosubstituierten Malonsäuremonoester oder deren Salzen der obigen allgemeinen Formel I bereitgestellt, das durch eine enzymatische Teilhydrolyse von α-monosubstituierten Malonsäurediestern der allgemeinen Formel II: 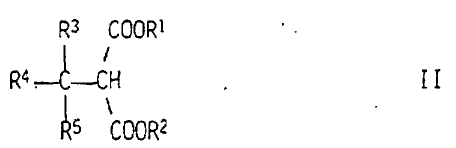 gekennzeichnet ist, in der R¹, R³, R⁴ und R⁵ die für die Formel I angegebene Bedeutung haben und R² ebenfalls einen Kohlenwasserstoffrest bezeichnet, der von R¹ verschieden sein kann.

Das Verfahren nach der Erfindung ergibt die α-monosubstituierten Malonsäuremonoester I und deren Salze mit guten Ausbeuten und hoher Enantioselektivität, ausgehend von den entsprechenden α-monosubstituierten Malonsäurediestern II, die wohlfeil und auch in technischen Mengen gut zugänglich sind. Der tertiäre Kohlenwasserstoffrest des α-monosubstituierten Malonsäurediesters II mit seinem quatären. an das α-ständige Kohlenstoffatom des Malonsäurediesters II gebundenen Kohlenstoffatom ermöglicht offenbar eine enantioselektive Hydrolyse nur der einen Carbonestergruppe. Die enantiomerenangereicherten α-monosubstituierten Malonsäuremonoester sind über einen weiten pH-Bereich konfigurationsstabil. Dies ist überraschend, weil nach der Literatur (T.Kitazume et al.. loc. cit.) monosubstituierte Malonsäurediester bei der enzymatischen Verseifung racemische Produkte ergeben.

In den bevorzugten α-monosubstituierten Malonsäuremonoestern I, und dementsprechend auch in den als Ausgangsstoffe bevorzugten α-monosubstituierten Malonsäurediestern II, bedeutet R¹ einen niederen Alkylrest mit 1 bis 4, insbesondere 1 oder 2 Kohlenstoffatomen oder den Benzylrest, und sind R³, R⁴ sowie R⁵ gleich oder verschieden und bedeuten Alkyl-, Alkenyl-, Aryl-, Alkaryl- oder Aralkylreste mit bis zu 10 Kohlenstoffatomen. Wenn jeweils zwei der Reste R³, R⁴ und R⁵ mit dem quartären Kohlenstoffatom, das sie substituieren, einen carbocyclischen Ring bilden. so hat dieser Ring bevorzugt gesättigte Kohlenwasserstoffstruktur und 5 bis 12, insbesondere 5 oder 6 Ringglieder. H in der Formel I steht bevorzugt für Wasserstoff, ein Alkali- oder Erdalkalimetalläquivalent oder ein gegebenenfalls alkylsubstituiertes Ammoniumion. R² in der Formel II hat dieselbe bevorzugte Bedeutung wie R¹ und kann von R¹ verschieden sein.

Wenn alle drei Substituenten R³, R⁴ und R⁵ verschieden sind, haben die Malonsäureester ein Chiralitätszentrum. Die α-monosubstituierten Malonsäurediester II können also als Racemat oder enantiomerenrein auftreten. Beide eignen sich als Ausgangsstoffe für das Verfahren nach der Erfindung. Im Falle der Racemate verläuft die enzymatische Teilhydrolyse als kinetische Racematspaltung, wodurch das Ausmaß des nach dem Verfahren der Erfindung erzielbaren Enantiomerenüberschusses beeinflußt wird. Dieser beträgt im allgemeinen, je nach den Substituenten R³, R⁴ und R⁵, von 80 bis >99%. Die α-monosubstituierten Malonsäurediester lassen sich aus den unsubstituierten Malonsäurediestern durch Umsetzung mit den entsprechenden Elektrophilen in bekannter Weise herstellen.

Von den geeigneten Malonsäurediestern II seien z.B. α-tert.-Butylmalonsäurediethylester. α-tert.-Butylmalonsäuredimethylester, α-tert.-Pentylmalonsäuredibenzylester. α-tert.-Pentylmalonsäuredimethylester, α-(1-Methyl-1-phenylethyl)-malonsäuredimethylester. α-(1-Methyl-1-phenyl-n-propyl)-malonsaurediethylester. α-(1-Ethyl-1-phenylethyl)-malonsäuredibutylester. α-(1-Methylcyclohexyl)-malonsäurediethylester und α-(1,1-Dimethylprop-2-enyl)-malonsäurediethylester genannt.

Als Katalysatoren für die enzymatische Teilhydrolyse sind alle bekannten Enzyme verwendbar, die in wässerigem Medium eine Hydrolyse von Carbonestergruppen bewirken. Dafür eignen sich alle handelsüblichen esterspaltenden Enzyme, zu denen man Esterasen, Lipasen und Proteasen zählt. Sie können in kristalliner Form. in wäßriger Suspension oder auf einem Träger fixiert eingesetzt werden. Von den geeigneten Enzymen seien beispielsweise die bereits erwähnte Schweineleberesterase. Lipase aus Candida cylindracea. α-Chymotrypsin. Papain aus Carica papaya und Lipase aus Schweinepankreas erwähnt.

Zur Durchführung des Verfahrens nach der Erfindung suspendiert man den α-monosubstituierten Malonsäurediester II zweckmäßig in Wasser, einer wäßrigen Lösung oder Pufferlösung, die den optimalen pH-Wert einzustellen gestattet. Geeignete Puffer sind z.B. Phosphatpuffer. Citratpuffer sowie Tris(-hydroxymethyl)aminomethan (kurz "Tris" genannt). Sie werden im allgemeinen in Konzentrationen von 0.01 bis 3 m verwendet und zweckmäßig in der 2 bis 50-fachen Gewichtsmenge. bezogen auf den α-monosubstituierten Malonsäurediester II. eingesetzt. Der Zusatz eines Lösungsmittels. beispielsweise in Mengen von bis zu etwa 40 Gewichtsprozent, bezogen auf die wässerige Pufferlösung, ist in manchen Fällen hilfreich. Geeignete Lösungsmittel sind z.B. Ethanol. Tetrahydrofuran. Dimethylsulfoxid. N-Methylpyrrolidon und Acetonitril.

Die Teilhydrolyse nach der Erfindung findet unter den für enzymatische Reaktionen typischen milden Bedingungen statt. Der pH-Wert wird vorteilhaft durch Zugabe einer Hydroxylionen liefernden Lösung oder Suspension eines basischen Stoffs im Bereich von etwa 4 bis etwa 9 gehalten. Der optimale pH-Wert hängt wesentlich von dem verwendeten Enzym ab und läßt sich durch orientierende Versuche unschwer ermitteln. Geeignete basische Stoffe sind z.B. Metallhydroxide oder gegebenenfalls substituiertes Ammoniumhydroxid. Bevorzugt werden verdünnte. d.h. 0.05 bis 2 n Lösungen von Alkali- oder Erdalkalihydroxiden, Alkalicarbonaten und/oder Alkalihydrogencarbonaten. Weiterhin werden entsprechend verdünnte wässerige Lösungen von Ammoniak sowie von primären, sekundären oder tertiären Alkylaminen, wie Methylamin. Diethylamin oder Tributylamin, als Hydroxylionen liefernde Lösungen bevorzugt. Die Hydroxylionen werden in molaren Mengen benötigt, so daß am Ende der Reaktion die α-monosubstituierten Malonsäuremonoester I in Form ihrer entsprechenden Salze gelöst vorliegen.

Die Teilhydrolyse nach der Erfindung findet im allgemeinen unter Atmosphärendruck statt, man kann aber auch erhöhte oder verminderte Drücke anwenden, z.B. 0.7 bis 2 bar. Unter den angegebenen Bedingungen bleibt die Hydrolyse eine Teilhydrolyse, d.h. die zweite Carbonestergruppe wird nicht angegriffen.

Das Verfahren kann diskontinuierlich oder kontinuierlich durchgeführt werden. Bei der diskontinuierlichen Arbeitsweise kann man z.B. den α-monosubstituierten Malonsäurediester, die Pufferlösung, das Enzym und gegebenenfalls ein Lösungsmittel in einem Rührgefäß vorlegen, das Gemisch auf die gewünschte Temperatur bringen und durch Rühren für eine gute Durchmischung sorgen. Bei dieser Arbeitsweise werden Mengen im Bereich von Nanomolen bis etwa 0.5 mol α-monosubstituierter Malonsäurediester II pro mg Enzym eingesetzt. Der pH-Wert des Gemisches wird überwacht und durch Zugabe einer Hydroxylgruppen liefernden Lösung in dem angegebenen Bereich gehalten. Zur Aufarbeitung kann man das Reaktionsgemisch z.B. mit Schwefel- oder Salzsäure ansäuern, feste Bestandteile. darunter das Enzym, abfiltrieren und durch Extraktion der sauren Lösung mit einem wasserunlöslichen Lösungsmittel, wie Diethylether, und Verdampfen des Lösungsmittels aus der zuvor zweckmäßig getrockneten Etherlösung den α-monosubstituierten Malonsäuremonoester in Substanz gewinnen. Alternativ kann man das Enzym auch durch Zentrifugieren oder mittels eines Membranverfahrens abtrennen und danach die Lösung ansäuern.

Den Enantiomerenüberschuß kann man in bekannter Weise bestimmen, z.B. indem man den α-monosubstituierten Malonsäuremonoester in das Säurechlorid überführt, beispielsweise mittels Cyanurchlorid, dieses mit einem optisch aktiven Amin, beispielsweise mit (S)-Phenylethylamin, zum Carbonsäureamid umsetzt, das Verhältnis der Isomeren durch quantitative gaschromatographische Analyse bestimmt und daraus den Enantiomerenüberschuß errechnet. Durch herkömmliche enantioselektive oder diastereoselektive Kristallisationsverfahren (E.L.Eliel, S.H.Wilen und L.N.Manden. Stereochemistry of Organic Compounds. John Wiley, 1994) kann man aus den enantiomerenangereicherten Produkten die enantiomerenreinen α-monosubstituierten Malonsäuremonoester gewinnen.

Wenn man das wie beschrieben aus dem Reaktionsgemisch abgetrennte oder ein enantiomerenreines Produkt in der wässerigen Lösung eines basischen Stoffs, wie zuvor erläutert, lost. erhält man das entsprechende Salz des α-monosubstituierten Malonsäuremonoesters 1. das durch Eindampfen der Lösung, zweckmäßig im Vakuum. als Festprodukt isoliert oder in der Lösung weiter umgesetzt werden kann.

Das Verfahren nach der Erfindung kann kontinuierlich durchgeführt werden. indem man z.B. nach bekannten Verfahren in einem Enzym-Membran-Reaktor arbeitet oder ein Gemisch aus der Pufferlosung, dem α-monosubstituierten Malonsäurediester II und gegebenenfalls einem Lösungsmittel unter ständiger Kontrolle des pH-Wertes über ein in einem Reaktionsrohr fest angeordnetes immobilisiertes Enzym rieseln läßt und den Durchlauf aufarbeitet, wie zuvor beschrieben, wobei natürlich die Abtrennung des Enzyms entfällt.

Die folgenden Beispiele sollen die Erfindung weiter erläutern, aber nicht ihren Umfang begrenzen, wie er in den Patentansprüchen definiert ist.

### Beispiele

### Beispiel 1

In einem Gemisch aus 10 ml 0.1 m wässeriger Kaliumdihydrogenphosphat-Lösung und 15 ml 0,1 m wässeriger Dinatriumhydrogenphosphat-Lösung werden 3.0 g (13.9 mmol) α-tert.-Butylmalonsäurediethylester suspendiert. Zu der Suspension werden 1.3 ml Chirazyme E1 (Boehringer Mannheim GmbH) gegeben. Man überwacht den pH-Wert und dosiert langsam 1 n Natronlauge zu der gerührten und auf Raumtemperatur gehaltenen Suspension. so daß der pH-Wert im Bereich von 6 bis 9 liegt. Innerhalb von 48 Stunden werden 13 ml (13 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 2.35 g (12.5 mmol) α-tert.-Butylmalonsäuremonoethylester, entsprechend einer Ausbeute von 90 % der Theorie. Der Enantiomerenüberschuß beträgt 96%.

### Beispiel 2

In einem Gemisch aus 5 ml 0.1 m wässeriger Kaliumdihydrogenphosphat-Lösung und 7,5 ml 0.1 m wässeriger Dinatriumhydrogenphosphat-Lösung werden 533 mg (2.83 mmol) α-tert.-Butylmalonsäurediethylester suspendiert. Zu der Suspension werden 0,3 ml Schweineleberesterase (Boehringer Mannheim GmbH. Katalog-Nr. 104698) gegeben. Man überwacht den pH-Wert und dosiert langsam 1 n Natronlauge zu der gerührten und auf Raumtemperatur gehaltenen Suspension, so daß der pH-Wert im Bereich von 6 bis 9 liegt. Innerhalb von 24 Stunden werden 2.9 ml (2.9 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 432 mg (2.48 mmol) a-tert.-Butylmalonsäuremonoethylester. entsprechend einer Ausbeute von 85 % der Theorie. Der Enantiomerenüberschuß beträgt 89%.

### Beispiel 3

In einem Gemisch aus 5 ml 0.1 m wässeriger Kaliumdihydrogenphosphat-Lösung und 7.5 ml 0.1 m wässeriger Dinatriumhydrogenphosphat-Lösung werden 500 mg (2,17 mmol) α-tert.-Pentylmalonsäurediethylester suspendiert. Zu der Suspension werden 0.3 ml Schweineleberesterase (Boehringer Mannheim GmbH. Katalog-Nr. 104698) gegeben. Man überwacht den pH-Wert und dosiert langsam 1 n Natronlauge zu der gerührten und auf Raumtemperatur gehaltenen Suspension, so daß der pH-Wert im Bereich von 6 bis 9 liegt. Innerhalb von 24 Stunden werden 2.2 ml (2.2 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 334 mg (1.65 mmol) α-tert.-Pentylmalonsäuremonoethylester. entsprechend einer Ausbeute von 75 % der Theorie. Der Enantiomerenüberschuß beträgt 82%.

### Beispiel 4

In einem Gemisch aus 5 ml 0.1 m wässeriger Kaliumdihydrogenphosphat-Lösung und 7,5 ml 0.1 m wässeriger Dinatriumhydrogenphosphat-Lösung. werden 454 mg (1.67 mmol) α-(1-Methyl-1-phenylethyl)-malonsäurediethylester suspendiert. Zu der Suspension werden 0.4 ml Schweineleberesterase (Boehringer Mannheim GmbH. Katalog-Nr 104698) gegeben. Man überwacht den pH-Wert und dosiert langsam 1 n Natronlauge zu der gerührten und auf Raumtemperatur gehaltenen Suspension, so daß der pH-Wert im Bereich von 6 bis 9 liegt. Innerhalb von 24 Stunden werden 1.7 ml (1.7 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 340 mg (1.35 mmol), α-(1-Methyl-1-phenylethyl)-malonsäuremonoethylester. entsprechend einer Ausbeute von 81 % der Theorie. Der Enantiomerenüberschuß beträgt 94%.

### Beispiel 5

In einem Gemisch aus 10 ml 0.1 m wässeriger Kaliumdihydrogenphosphatlösung und 15 ml 0.1 m wässeriger Dinatriumhydrogenphosphatlösung werden 533 mg (2.83 mmol) α-tert.Butylmalonsäuredimethylester suspendiert. Zu der Suspension werden 0.4 ml Schweineleberesterase (Boehringer Mannheim GmbH, Katalog-Nr. 104698) gegeben. Man überwacht den pH-Wert und dosiert langsam 1 n Natronlauge zu der gerührten und auf Raumtemperatur gehaltenen Suspension, so daß der pH-Wert im Bereich von 6 bis 9 liegt. Innerhalb von 30 Stunden werden 2.8 ml (2.8 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 432 mg (2.48 mmol) α-tert.Butyl-malonsäuremonomethylester, entsprechend einer Ausbeute von 87 % der Theorie. Der Enantiomerenüberschuß beträgt 89%.

### Beispiel 6

In einem Gemisch aus 10 ml 0,1 m wässeriger Kaliumdihydrogenphosphatlösung und 15 ml 0.1 m wässeriger Dinatriumhydrogenphosphatlösung werden 476 mg (1.9 mmol) α-(1-Methyl-1-phenylethyl)-malonsäuredimethylester suspendiert. Zu der Suspension werden 0.5 ml Schweineleberesterase (Boehringer Mannheim GmbH, Katalog-Nr. 104698) gegeben. Man überwacht den pH-Wert und dosiert langsam 1 n Natronlauge zu der gerührten und auf Raumtemperatur gehaltenen Suspension, so daß der pH-Wert im Bereich von 6 bis 9 liegt. Innerhalb von 30 Stunden werden 1,6 ml (1,6 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 305 mg (1,29 mmol) α-(1-Methyl-1-phenylethyl)-malonsäuremonomethylester. entsprechend einer Ausbeute von 68 % der Theorie. Der Enantiomerenüberschuß beträgt 90%.

### Beispiel 7

In einem Gemisch aus 10 ml 0,1 m wässeriger Kaliumdihydrogenphosphatlösung und 15 ml 0.1 m wässeriger Dinatriumhydrogenphosphatlösung werden 500 mg (1.9 mmol) α-(1-Methylcyclohexyl)-malonsäuredimethylester suspendiert. Zu der Suspension werden 0.5 ml Schweineleberesterase (Boehringer Mannheim GmbH. Katalog-Nr. 104698) gegeben. Man überwacht den pH-Wert und dosiert langsam 1 n Natronlauge zu der gerührten und auf Raumtemperatur gehaltenen Suspension, so daß der pH-Wert im Bereich von 6 bis 9 liegt. Innerhalb von 40 Stunden werden 1,9 ml (1,9 mmol) 1 n Natronlauge verbraucht. Dann wird das Reaktionsgemisch mit konzentrierter Salzsäure angesäuert (pH 1-2) und über Celite filtriert. Man extrahiert das Reaktionsprodukt mehrmals mit Diethylether und trocknet die Etherphase über Magnesiumsulfat. Nach dem Entfernen des Lösungsmittels verbleiben 400 mg (1.76 mmol) α-(1-Methylcyclohexyl)-malonsäuremonomethylester, entsprechend einer Ausbeute von 90 % der Theorie. Der Enantiomerenüberschuß beträgt 96%.

## Patentansprüche

1. Enantiomerenangereicherte, durch einen tertiären Kohlenwasserstoffrest α-monosubstituierte Malonsäuremonoester oder deren Salze der allgemeinen Formel I: 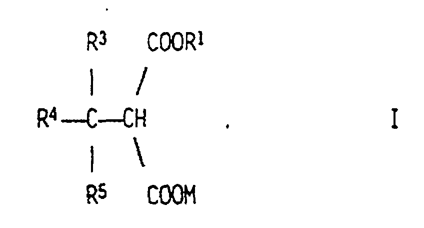 in der R¹, R³, R⁴ und R⁵ gleiche oder verschiedene Kohlenwasserstoffreste bedeuten, jeweils zwei der Reste R³, R⁴ und R⁵ zusammen mit dem quartären Kohlenstoffatom, das sie substituieren, auch einen carbocyclischen Ring bilden können und M Wasserstoff, ein Metalläquivalent oder ein gegebenenfalls substituiertes Ammoniumion bezeichnet.

2. Enantiomerenangereicherte, durch einen tertiären Kohlenwasserstoffrest α-monosubstituierte Malonsäuremonoester oder deren Salze der allgemeinen Formel I nach Anspruch 1, **dadurch gekennzeichnet, daß** R¹ einen Alkylrest mit 1 bis 4 Kohlenstoffatomen oder den Benzylrest bedeutet. R³. R⁴ und R⁵ gleiche oder verschiedene Alkyl-, Alkenyl-. Aryl-. Alkaryl- oder Aralkylreste mit bis zu 10. Kohlenstoffatomen bezeichnet, jeweils zwei der Reste R³, R⁴ und R⁵ zusammen mit dem quartären Kohlenstoffatom, das sie substituieren, auch einen carbocyclischen Ring mit 5 bis 12 Kohlenstoffatomen bilden können und M für Wasserstoff. ein Alkalimetall- oder Erdalkalimetalläquivalent oder ein gegebenenfalls alkylsubstituiertes Ammoniumion steht.

3. Verfahren zur Herstellung von enantiomerenangereicherten. durch einen tertiären Kohlenwasserstoffrest α-monosubstituierten Malonsäuremonoestern oder deren Salzen der allgemeinen Formel I: 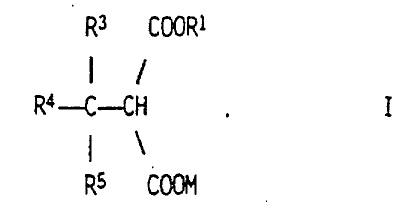 in der R¹, R³, R⁴ und R⁵ gleiche oder verschiedene Kohlenwasserstoffreste bedeuten, jeweils zwei der Reste R³, R⁴ und R⁵ zusammen mit dem quartären Kohlenstoffatom, das sie substituieren, auch einen carbocyclischen Ring bilden können und M Wasserstoff, ein Metalläquivalent oder ein gegebenenfalls substituiertes Ammoniumion bezeichnet.
**gekennzeichnet durch** eine enzymatische Teilhydrolyse von α-monosubstituierten Malonsäurediestern der allgemeinen Formel II: 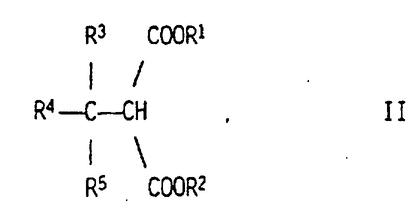 in der R¹, R³, R⁴ und R⁵ die für die Formel I angegebene Bedeutung haben und R² ebenfalls einen Kohlenwasserstoffrest bezeichnet, der von R¹ verschieden sein kann.

4. Verfahren zur Herstellung von enantiomerenangereicherten, durch einen tertiären Kohlenwasserstoffrest α-monosubstituierten Malonsäuremonoestern oder deren Salzen der allgemeinen Formel I nach Anspruch 3. **dadurch gekennzeichnet, daß** R¹ einen Alkylrest mit 1 bis 4 Kohlenstoffatomen oder den Benzylrest bedeutet. R³, R⁴ und R⁵ gleiche oder verschiedene Alkyl-, Alkenyl-, Aryl-, Alkaryl- oder Aralkylreste mit bis zu 10 Kohlenstoffatomen bezeichnet, jeweils zwei der Reste R³, R⁴ und R⁵ zusammen mit dem quartären Kohlenstoffatom, das sie substituieren, auch einen carbocyclischen Ring mit 5 bis 12 Kohlenstoffatomen bilden können und M für Wasserstoff, ein Alkalimetall- oder Erdalkalimetalläquivalent oder ein gegebenenfalls alkylsubstituiertes Ammonium-ion steht.

5. Verfahren nach Anspruch 3 oder 4, **dadurch gekennzeichnet, daß** handelsübliche Esterasen, Lipasen, Proteasen oder andere esterspaltende Enzyme als Katalysatoren für die Teilhydrolyse verwendet werden

6. Verfahren nach einem der Ansprüche 3 bis 5, **dadurch gekennzeichnet. daß** die enzymatische Teilhydrolyse in Wasser, einer wässerigen Lösung oder Pufferlösung durchgeführt und gegebenenfalls eine Hydroxylionen liefernde Lösung oder Suspension eines basischen Stoffs zugegeben wird, um einen für das jeweilige Enzym optimalen pH-Wert einzustellen.

7. Verfahren nach Anspruch 6, **dadurch gekennzeichnet. daß** der basische Stoff ein Alkali- oder Eralkalimetallhydroxid, ein Alkalicarbonat oder Alkalihydrogencarbonat oder gegebenenfalls alkylsubstituiertes Ammoniumhydroxid ist.

8. Verfahren nach einem der Ansprüche 3 bis 7. **dadurch gekennzeichnet, daß** ein wasserlösliches Lösungsmittel mitverwendet wird.

9. Verfahren nach einem der Ansprüche 3 bis 8, **dadurch gekennzeichnet. daß** die enzymatische Teilhydrolyse bei einer Temperatur von 15 bis 50°C, vorzugsweise von 20 bis 30°C, und unter einem Druck von 0.8 bar bis 2.0 bar durchgeführt wird.

10. Verfahren nach einem der Ansprüche 3 bis 9, **dadurch gekennzeichnet, daß** das Teilhydrolysegemisch zur Aufarbeitung angesäuert und der α-monosubstituierte Malonsäuremonoester I. gegebenenfalls nach Abtrennung von Feststoffen, mit einem wasserunlöslichen Lösungsmittel aus dem wässerigen Medium extrahiert wird.

## Claims

1. An enantiomer-enriched malonic acid monoester α-monosubstituted by a tertiary hydrocarbon radical, or a salt thereof, of the general formula I: 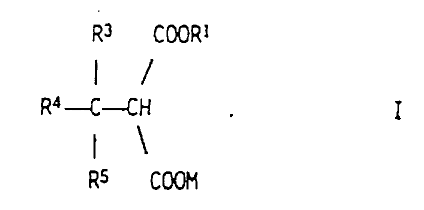 in which R¹, R³, R⁴ and R⁵ are identical or different hydrocarbon radicals, in each case two of the radicals R³, R⁴ and R⁵ together with the quaternary carbon atom which they substitute can also form a carbocyclic ring, and M is hydrogen, a metal equivalent or an optionally substituted ammonium ion.

2. An enantiomer-enriched malonic acid monoester α-monosubstituted by a tertiary hydrocarbon radical, or a salt thereof, of the general formula I according to claim 1, **characterized in that** R¹ is an alkyl radical having 1 to 4 carbon atoms or the benzyl radical, R³, R⁴ and R⁵ are identical or different alkyl, alkenyl, aryl, alkaryl or aralkyl radicals having up to 10 carbon atoms, in each case two of the radicals R³, R⁴ and R⁵ together with the quaternary carbon atom which they substitute can also form a carbocyclic ring having 5 to 12 carbon atoms, and M is hydrogen, an alkali metal equivalent or alkaline earth metal equivalent or an optionally alkyl-substituted ammonium ion.

3. A process for the preparation of enantiomer-enriched malonic acid monoesters α-substituted by a tertiary hydrocarbon radical, or salts thereof, of the general formula I: 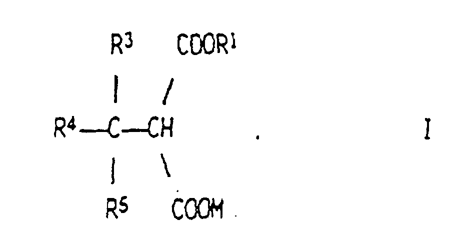 in which R¹, R³, R⁴ and R⁵ are identical or different hydrocarbon radicals, in each case two of the radicals R³, R⁴ and R⁵ together with the quaternary carbon atom which they substitute can also form a carbocyclic ring, and M is hydrogen, a metal equivalent or an optionally substituted ammonium ion, **characterized by** an enzymatic partial hydrolysis of α-monosubstituted malonic acid diesters of the general formula II: 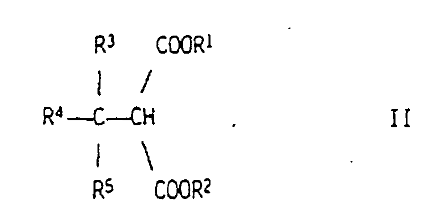 in which R¹, R³, R⁴ and R⁵ have the meanings given for formula I, and R² is likewise a hydrocarbon radical, which may be different from R¹.

4. A process for the preparation of enantiomer-enriched malonic acid monoesters α-monosubstituted by a tertiary hydrocarbon radical, or salts thereof, of the general formula I according to claim 3, **characterized in that** R¹ is an alkyl radical having 1 to 4 carbon atoms or the benzyl radical, R³, R⁴ and R⁵ are identical or different alkyl, alkenyl, aryl, alkaryl or aralkyl radicals having up to 10 carbon atoms, in each case two of the radicals R³, R⁴ and R⁵ together with the quaternary carbon atom which they substitute can also form a carbocyclic ring having 5 to 12 carbon atoms, and M is hydrogen, an alkali metal equivalent or alkaline earth metal equivalent or an optionally alkyl-substituted ammonium ion.

5. A process according to claim 3 or 4, **characterized in that** standard commercial esterases, lipases, proteases or other ester-cleaving enzymes are used as catalysts for the partial hydrolysis.

6. A process according to any of claims 3 to 5, **characterized in that** the enzymatic partial hydrolysis is carried out in water, an aqueous solution or buffer solution and, if desired, a hydroxyl-ion-supplying solution or suspension of a basic substance is added in order to establish a pH which is optimum for the respective enzyme.

7. A process according to claim 6, **characterized in that** the basic substance is an alkali metal hydroxide or alkaline earth metal hydroxide, an alkali metal carbonate or alkali metal hydrogencarbonate or optionally alkyl-substituted ammonium hydroxide.

8. A process according to any of claims 3 to 7, **characterized in that** a water-soluble solvent is co-used.

9. A process according to any of claims 3 to 8, **characterized in that** the enzymatic partial hydrolysis is carried out at a temperature of from 15 to 50°C, preferably from 20 to 30°C, and under a pressure of from 0.8 bar to 2.0 bar.

10. A process according to any of claims 3 to 9, **characterized in that** the partial hydrolysis mixture is acidified for work-up, and the α-monosubstituted malonic acid monoester I, if desired following removal of solids, is extracted from the aqueous medium with a water-insoluble solvent.

## Revendications

1. Monoesters d'acide malonique enrichis en énantiomères, monosubstitués en α par un reste d'hydrocarbure tertiaire, ou leurs sels, de formule générale : 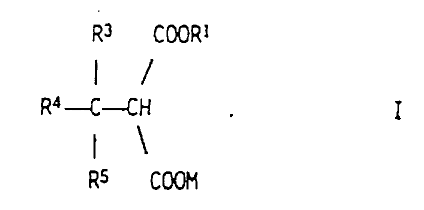 dans laquelle R¹, R³, R⁴ et R⁵ signifient des restes hydrocarbonés identiques ou différents, respectivement deux des restes R³, R⁴ et R⁵ conjointement avec l'atome de carbone quaternaire qu'ils substituent, peuvent former aussi un cycle carbocyclique et M désigne un hydrogène un équivalent métallique ou un ion ammonium éventuellement substitué.

2. Monoesters d'acide malonique enrichis en énantiomères, monosubstitués en α, par un reste d'hydrocarbure tertiaire ou leurs sels de formule générale I selon la revendication 1,
**caractérisés en ce que**
R¹ signifie un reste alkyle ayant de 1 à 4 atomes de carbone ou le reste benzyle, R³, R⁴ et R⁵ désignent des restes alkyle, alkényle, aryle, alkaryle, ou aralkyle ayant, jusqu'à 10 atomes de carbone, respectivement deux des restes R³, R⁴ et R⁵ ensemble avec l'atome de carbone quaternaire qu'ils substituent, peuvent former aussi un cycle carbocyclique ayant de 5 à 12 atomes de carbone, et M représente de l'hydrogène, un équivalent de métal alcalin ou alcalino-terreux ou un ion ammonium éventuellement substitué par un alkyle.

3. Procédé de préparation de monoesters d'acide malonique enrichis en énantiomères, monosubstitués en α, par un reste d'hydrocarbure tertiaire ou leurs sels de formule générale I, 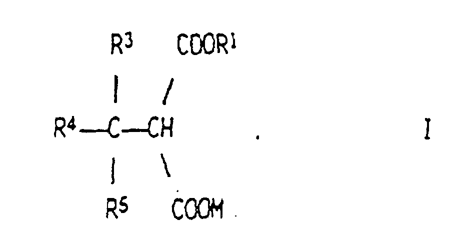 dans laquelle R¹, R³, R⁴ et R⁵ signifient des restes hydrocarbonés identiques ou différents, respectivement deux des restes R³, R⁴ et R⁵ ensemble avec l'atome de carbone quaternaire qu'ils substituent, peuvent former aussi un cycle carbocyclique, et M désigne un hydrogene, un équivalent métallique ou un ion ammonium éventuellement substitué,
**caractérisé par**
une hydrolyse partielle enzymatique de diesters d'acide malonique monosubstitués en α, de formule générale II, 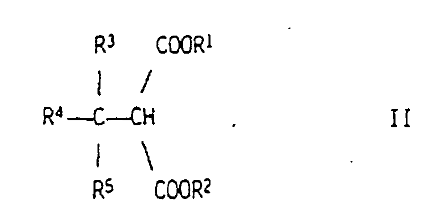 dans laquelle R¹, R³, R⁴ et R⁵ ont la signification indiquée pour la formule I et R² également désigne un reste d'hydrocarbure qui peut être différent de R¹.

4. Procédé de préparation de monoesters d'acide malonique enrichis en énantiomères, monosubstitués en α, par un reste d'hydrocarbure tertiaire ou leurs sels de formule générale I selon la revendication 3,
**caractérisé en ce que**
R¹ signifie un reste alkyle ayant de 1 à 4 atomes de carbone ou le reste benzyle, R³, R⁴ et R⁵ désignent des restes alkyle, alkényle, aryle, alkaryle ou aralkyle, identiques ou différents, ayant jusqu'à 10 atomes de carbone, respectivement deux des restes R³, R⁴ et R⁵ ensemble avec l'atome de carbone quaternaire qu'ils substituent, peuvent former aussi un cycle carbocyclique ayant de 5 à 12 atomes de carbone, et M représente un hydrogène, un équivalent de métal alcalin ou alcalino-terreux ou un ion ammonium éventuellement substitué par un alkyle.

5. Procédé selon l'une quelconque des revendications 3 ou 4,
**caractérisé en ce qu'**
on utilise des estérases, des lipases, des protéases ou d'autres enzymes qui coupent les esters, commerciaux, en tant que catalyseurs pour l'hydrolyse partielle.

6. Procédé selon l'une quelconque des revendications 3 à 5,
**caractérisé en ce que**
l'hydrolyse partielle enzymatique est effectuée dans l'eau, dans une solution aqueuse ou une solution tampon, et une solution qui apporte des ions hydroxyle ou une suspension d'une substance basique est éventuellement ajoutée afin d'ajuster une valeur de pH optimale pour l'enzyme respectif.

7. Procédé selon la revendication 6,
**caractérisé en ce que**
la substance basique est un hydroxyde de métal alcalin ou alcalino-terreux, un carbonate de métal alcalin ou un hydrogénocarbonate de métal alcalin ou un hydroxyde d'ammonium éventuellement substitué par un alkyle.

8. Procédé selon l'une quelconque des revendications 3 à 7,
**caractérisé en ce qu'**
on utilise conjointement un solvant soluble dans l'eau.

9. Procédé selon l'une quelconque des revendications 3 à 8,
**caractérisé en ce qu'**
on exécute l'hydrolyse partielle enzymatique à une température allant de 15 à 50°C, de préférence de 20 à 30°C, et sous une pression allant de 0,8 à 2,0 bars.

10. Procédé selon l'une quelconque des revendications 3 à 9,
**caractérisé en ce qu'**
on acidifie le mélange d'hydrolyse partielle en vue de la purification, et on extrait le monoester d'acide malonique monosubstitué en α (I), le cas échéant après séparation des matières solides, du milieu aqueux avec un solvant insoluble dans l'eau.
